# EUROPEAN PATENT APPLICATION

(11) **EP 3 763 375 A1**
(43) Date of publication of application: **13.01.2021**
(21) Application number: 19763545.1
(22) Date of filing: 05.03.2019
(51) Int. Cl.: A61K 35/28, A61K 35/15, A61P 1/00, A61P 1/18, A61P 3/10, A61P 9/00, A61P 9/04, A61P 9/10, A61P 11/00, A61P 13/12, A61P 25/00, A61P 25/28, G01N 33/15, G01N 33/48

(54) **CELL PREPARATION FOR TREATMENT AND/OR PREVENTION OF ISCHEMIC DISEASE, AND METHOD FOR SCREENING CELL PREPARATION**

(30) Priority: 08.03.2018 JP 2018041841
(71) Applicant: Foundation for Biomedical Research and Innovation at Kobe, Kobe-shi Hyogo 650-0047 (JP)
(72) Inventor: TAGUCHI, Akihiko, Kobe-shi, Hyogo 650-0047 (JP)
(74) Representative: J A Kemp LLP
(86) International application number: PCT/JP2019/008701
(87) International publication number: WO 2019/172279

(57) **Abstract**

The present invention provides a cell preparation in which a proportion of leukocyte in a normal form and an undesired component containing at least one selected from the group consisting of erythrocyte, deformed cell, platelet and aggregate (undesired component count/leukocyte count) is not more than a predetermined value. The aforementioned predetermined value is preferably 37.0%. In addition, the cell preparation of the present invention can be utilized for the treatment of ischemic diseases such as cerebral infarction, myocardial infarction, limb ischemia, renal infarction, pulmonary infarction, splenic infarction, the intestinal infarction, Buerger disease, cerebrovascular dementia, diabetic nephropathy microangiopathy, diabetic cardiac failure and the like.

## Description

### [Technical Field]

The present invention relates to a cell preparation for treating and/or preventing ischemic diseases, and a screening method for the cell preparation.

### [Background Art]

An ischemic disease is a disease in which ischemia of an organ occurs due to obstruction or stenosis of an artery feeding the organ, and tissues become necrotic or dysfunctional due to lack of oxygen or nutrition, and includes various diseases such as myocardial infarction, limb ischemia, cerebral infarction, renal infarction, pulmonary infarction, splenic infarction, intestinal infarction, cerebrovascular dementia, and the like.

Protection/activation of the microvascular network is important for functional recovery of tissues damaged by ischemic disease. Recently, it has been suggested that cell transplantation is effective for the functional recovery. Non-patent document 1 in which an animal model of ischemic disease was used describes that administration of myeloid stem cells obtained by a specific gravity centrifugation method for myocardial infarction increases vascular density of microvascular network and improves cardiac function. Non-patent document 2 in which an animal model of ischemic disease was used describes that administration of myeloid stem cells obtained by a specific gravity centrifugation method for limb ischemia increases vascular density of microvascular network of the four limbs and improves ischemia symptoms. Non-patent document 3 in which an animal model of ischemic disease was used describes that administration of myeloid stem cells obtained by a specific gravity centrifugation method for cerebral infarction increases vascular density of microvascular network of the brain and improves neurological function.

On the other hand, it is known that, in patients with ischemic disease, administration of myeloid stem cells obtained by a specific gravity centrifugation method highly often fails to show any therapeutic effect. Non-patent document 4 relating to clinical trial for patients with ischemic disease shows that administration of myeloid stem cells obtained by a specific gravity centrifugation method to patients with myocardial infarction does not show any therapeutic effects. Non-patent document 5 points out that erythrocytes mixed in the administered cells may inhibit the therapeutic effect. In the clinical trial of non-patent document 4 performed based on that hypothesis, it has been shown that the administration of myeloid stem cells obtained by the specific gravity centrifugation method shows no clinical therapeutic effect even when erythrocyte contamination is prevented. In non-patent document 6 relating to clinical trial in patients with ischemic disease, in the administration of myeloid stem cells obtained by the specific gravity centrifugation method to patients with limb ischemia, it has been reported that amputation of ischemic limb due to exacerbation of ischemia or sudden death occurred in all cases of patients with arteriosclerotic limb ischemia after administration of myeloid stem cells (cases 2, 3, 4 and 6 in the document). Non-patent document 7 relating to clinical trial in patients with ischemic disease shows that administration of myeloid stem cells obtained by the specific gravity centrifugation method to patients with cerebral infarction does not have any clinical therapeutic effect.

As described above, myeloid stem cells obtained by a specific gravity centrifugation method often shows remarkable effects in animal experiments; however, the administration of myeloid stem cells obtained by the specific gravity centrifugation method to patients with ischemic disease highly frequently results in the development of non-responders who do not show any therapeutic effect. From the above, it is highly important to supply cells for treatment that show sufficient effectiveness for more patients.

### [Document List]

### [non-patent document]

non-patent document 1: Autologous transplantation of bone marrow mononuclear cells improved heart function after myocardial infarction. Lin et al. Acta Pharmacol Sin 2004 Jul; 25 (7): 876-886
non-patent document 2: Toward a mouse model of hind limb ischemia to test therapeutic angiogenesis. Brenes RA, et al. J Vasc Surg. 2012 Dec; 56(6):1669-1679;
non-patent document 3: Bone marrow mononuclear cells promote proliferation of endogenous neural stem cells through vascular niches after cerebral infarction. Nakano-Doi et al. Stem Cells. 2010 Jul; 28(7):1292-302.
non-patent document 4: Effect of the use and timing of bone marrow mononuclear cell delivery on left ventricular function after acute myocardial infarction: the TIME randomized trial. Traverse JH, et al. JAMA. 2012 Dec 12; 308(22):2380-2389. non-patent document 5: Erythrocyte contamination of the final cell product impairs the efficacy of autologous bone marrow mononuclear cell therapy. Assmus B, et al. J Am Coll Cardiol. 2010 Mar 30; 55(13):1385-1394.
non-patent document 6: Safety and efficacy of autologous progenitor cell transplantation for therapeutic angiogenesis in patients with critical limb ischemia. Kajiguchi M, et al. Circ J. 2007 Feb; 71(2):196-201.
non-patent document 7: Intravenous autologous bone marrow mononuclear stem cell therapy for ischemic stroke: a multicentric, randomized trial. Prasad K, Stroke. 2014 Dec; 45(12):3618-3624.

### [Summary of Invention]

### [Technical Problem]

In basic studies using an animal model of ischemic disease, administration of myeloid stem cells obtained by the specific gravity centrifugation method affords a therapeutic effect, whereas administration of myeloid stem cells obtained by the specific gravity centrifugation method to patients with ischemic disease is highly frequently associated with the presence of non-responders who do not show any therapeutic effect. The present invention aims to provide a cell preparation having a sufficient therapeutic effect in medical treatments targeting actual patients with ischemic disease, rather than bone marrow-derived stem cells effective in basic research using an animal model of ischemic disease. In addition, the present invention aims to provide a method for screening for a cell preparation having a sufficient therapeutic effect in medical treatments targeting actual patients with ischemic disease.

### [Solution to Problem]

The cell preparation of the present invention is characterized in that a proportion of leukocyte in a normal form and an undesired component containing at least one selected from the group consisting of erythrocyte, deformed cell, platelet and aggregate (undesired component count/leukocyte count) is not more than a predetermined value.

In addition, the screening method of the cell preparation of the present invention is characterized in that it includes a step of screening for one in which a proportion of leukocyte in a normal form and an undesired component containing at least one selected from the group consisting of erythrocyte, deformed cell, platelet and aggregate (undesired component count/leukocyte count) is not more than a predetermined value.

### [Advantageous Effects of Invention]

According to the present invention, it is possible to provide a cell preparation for treatment and/or prevention that exhibits a sufficient effect on patients with ischemic disease.

### [Brief Description of Drawings]

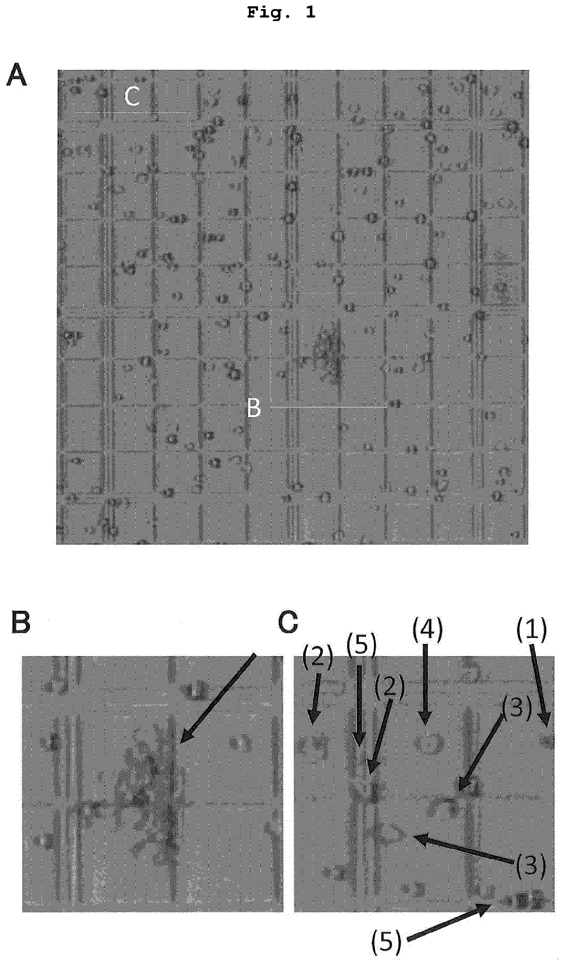

Fig. 1 A: A phase contrast micrograph of the administered cell suspension in Case 1. White frames B and C indicate the places of Figs. 1B and 1C, respectively. B: Aggregate considered to be formed by the fibrin mesh and the like was observed. C: When the phase contrast microscope was moved up and down for microscopic examination, a large number of apparently deformed cells and the like were observed, which were not leukocytes expected to be therapeutically effective. In (1), the cytoplasm is condensed due to a cause considered to be apoptosis, and the cell is apparently different from leukocytes having a cell function and expected to be therapeutically effective. In (2), the shape of the entire cell has already begun to disintegrate and identification of the cell type is difficult; it is a cell apparently different from leukocytes expected to be therapeutically effective. In (3), a part of the cell membrane has already broken; it is a cell apparently different from leukocytes expected to be therapeutically effective. (4) is considered to be erythrocyte; it is a cell apparently different from leukocytes expected to be therapeutically effective. (5) is considered to be platelet; it is apparently different from leukocytes expected to be therapeutically effective.
Fig. 2 illustrates how to count leukocyte and the like. As shown in the Figure, twelve squares with a side of 0.2 mm were randomly selected as fields, and the numbers of leukocytes, undesired cells and platelets present therein were respectively counted.
Fig. 3 is a phase contrast micrograph of the administered cell suspension in Case 2. Aggregate as seen in Case 1 was not observed, and the contamination with cells/platelets other than the cells expected to be therapeutically effective was clearly smaller than that in Case 1.
Fig. 4 is a phase contrast micrograph of the administered cell suspension in Case 3. Aggregate as seen in Case 1 was not observed, and the contamination with cells/platelets other than the cells expected to be therapeutically effective was clearly smaller than that in Case 1.
Fig. 5 shows the contamination proportion of undesired cells to desired leukocytes in each case. Compared with Case 1 in which a therapeutic effect was not observed, contamination with undesired cells was statistically significantly small in Cases 2 and 3 in which a therapeutic effect was observed.
Fig. 6 shows the contamination proportion of platelets to desired leukocytes in each case. Compared with Case 1 in which a therapeutic effect was not observed, contamination with platelets was statistically significantly small in Cases 2 and 3 in which a therapeutic effect was observed.

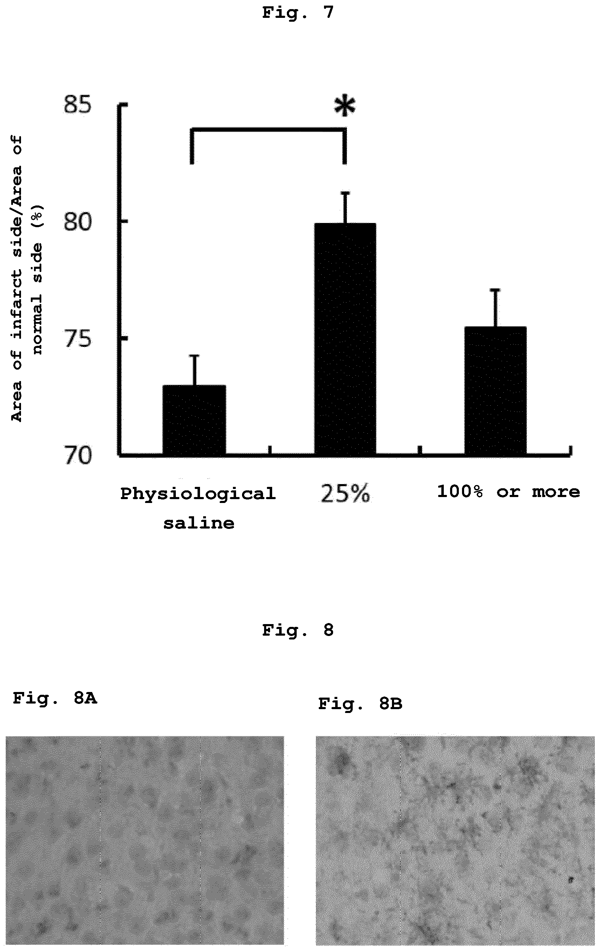

Fig. 7 shows the demonstration of a brain regeneration promoting effect of a leukocyte suspension depending on the proportion of undesired components. Comparison of [area of brain on which cerebral infarction was created]/[area of normal side on which cerebral infarction was not created] (area of infarct side/area of normal side (%)) 1 month after administration of leukocyte suspensions with different proportions of undesired components. Compared to the saline administration group (physiological saline solution), the group (25%) administered with the leukocyte suspension in which the undesired component count/leukocyte count was 25% showed a statistically significant brain regeneration promoting effect. On the other hand, the group (100% or more) administered with the leukocyte suspension in which the undesired component count/leukocyte count was 100% or more did not show a significant therapeutic effect.
Fig. 8 shows a brain regeneration promoting effect and an influence of contamination of extracellular components on an inflammatory response in the ischemic periphery region (outside the cerebral infarct area). A: An image in which ischemic peripheral region of cerebral infarction model mouse administered with the leukocyte suspension in which the undesired component count/leukocyte count was 25% was stained with an anti-CD11b antibody. B: An image in which ischemic peripheral region of individual administered with a leukocyte suspension in which the undesired component count/leukocyte count was 100% or more was stained with an anti-CD11b antibody. In the individual administered with a leukocyte suspension with a large proportion of contamination with undesired components, it is clear that CD11b-positive microglia/macrophages are activated and inflammation is induced.
Fig. 9 shows an inflammatory response in the ischemic peripheral region (outside the cerebral infarct area) of cerebral infarction model mouse administered with a leukocyte suspension in which the undesired component count/leukocyte count was 35%. It is clear that a leukocyte suspension in which the undesired component count/leukocyte count was 35% did not induce an inflammatory response.

### [Description of Embodiments]

The embodiment of the present invention is specifically explained in the following by referring to the attached Figures. The embodiment is intended to facilitate understanding of the principle of the present invention. The scope of the present invention is not limited to the following embodiment and other embodiments in which those skilled in the have appropriately substituted the configurations of the following embodiments are also included in the scope of the present invention.

For example, the methods for removing erythrocytes from bone marrow fluid mainly include two methods of a mechanical method using instrument and manual work. Manual work involves less erythrocyte contamination; however, mechanical methods using instruments are most commonly used. As mentioned above, the clinical therapeutic effect of the administration of myeloid stem cells that prevent erythrocyte contamination is not sufficient.

The present inventor obtained a surprising new finding in the administration of bone marrow-derived stem cells to patients with ischemic disease that, while the main desired component for revascularization is a mononuclear cell, the therapeutic effect is markedly attenuated when a large amount of undesired components are mixed in the cell suspension, and completed the present invention based on such fact.

Leukocytes are classified into granulocytes and mononuclear cells and immature cells that differentiate into them (hematopoietic stem cells, progenitor cells, lymphoblasts, monoblasts, myeloblasts, etc.). In the present specification, the desired component is a leukocyte in a normal form, that is, a leukocyte excluding cells in which condensation of whole cells is observed due to a cause considered to be apoptosis, cells in which the shape of the whole cell is disintegrated and identification of the cell type is difficult, and cells in which a part or all of the cell membrane is disrupted. Preferably, the desired component is a population of leukocytes (preferably mononuclear cell) in a normal form including hematopoietic stem cells. The undesired component is a component containing at least one selected from the group consisting of (a) an undesired cell, (b) a platelet, and (c) an aggregate. The undesired component may be a mixture of erythrocyte, deformed cell, platelet and aggregate. The undesired cell is (i) erythrocyte and/or (ii) a deformed cell of leukocyte or erythrocyte. The aggregate is a clumped object constituted of fibrin, and activated platelet and/or degenerated blood cell bound to the fibrin. In the present specification, both the "myeloid stem cell" and the "bone marrow-derived stem cell" mean bone marrow-derived hematopoietic stem cells.

That is, the cell preparation of the present invention means a cell preparation containing hematopoietic stem cells, which is characterized in that a proportion of leukocyte in a normal form and an undesired component (undesired component count/leukocyte count) is not more than a predetermined value, and the leukocyte count also contains the number of hematopoietic stem cells. The hematopoietic stem cell means CD34 positive cell, and the derivation of the cell (e.g., derived from bone marrow, derived from cord blood) is not particularly limited.

While the basis is shown in the Examples described below, the proportion of leukocytes in a normal form and undesired components is preferably undesired component count/leukocyte count ≤35.0%. The undesired component count/leukocyte count ≤20.0% is more preferable, and undesired component count/leukocyte count ≤14.0% is further preferable. The undesired component count/leukocyte count ≤37.0%, 25.0%, 12.0%, 10.6%, or 8.3% is also preferable. Among the undesired components that are inevitably mixed in the cell preparation, especially when aggregates are mixed, the therapeutic effect is remarkably attenuated. Thus, it is preferable that aggregates do not exist as much as possible in the undesired components. For example, aggregate count/leukocyte count ≤1.0% is preferable. In the aforementioned Examples, a cell preparation containing hematopoietic stem cells derived from bone marrow was used. It is expected that the therapeutic effect will be attenuated due to contamination with the undesired components even when a cell preparation containing hematopoietic stem cells other than the hematopoietic stem cells derived from bone marrow, for example, hematopoietic stem cells derived from cord blood. Thus, the above-mentioned ratios of the undesired component count/leukocyte count are also applicable to cell preparations containing hematopoietic stem cells other than the hematopoietic stem cells derived from bone marrow. Even when granulocytes are contained in the leukocyte cells obtained in the below-mentioned Examples, the number thereof is considered to be sufficiently smaller than the number of mononuclear cells. Also, the desired component that exerts a therapeutic effect is considered to be primarily mononuclear cells including hematopoietic stem cells. Therefore, the number of leukocytes in the above-mentioned proportions can be replaced with the number of mononuclear cells including hematopoietic stem cells.

The cell preparation of the present invention is used for the treatment and/or prevention of ischemic diseases. In the present specification, the "treatment" includes healing symptoms, improving symptoms, and suppressing the progression of symptoms. On the other hand, the "prevention" includes suppressing and delaying the onset of diseases, and also includes preventing not only becoming ill but also recurrence of the disease after treatment.

In the below-mentioned Examples, it is demonstrated that the cell preparation of the present invention promotes, as at least one effect, regeneration of tissue damage by reducing the inflammatory response in the ischemia peripheral region in cerebral ischemia, which response is detrimental to the regeneration process of tissue damage. The cell preparation of the present invention can be effective in ischemic diseases in general since it is known that inflammatory response is induced in the ischemic area not only in cerebral ischemia but also ischemic diseases in general. Therefore, the cell preparation of the present invention is for ischemic diseases, and the target disease includes, for example, cerebral infarction, myocardial infarction, limb ischemia, renal infarction, pulmonary infarction, splenic infarction, intestinal infarction, Buerger's disease, cerebrovascular dementia, diabetic nephropathy microangiopathy, diabetic heart failure, and the like. The present invention also includes a method for treating and/or preventing the above-mentioned ischemic diseases which includes administering the cell preparation of the present invention to mammals (target animal in the treatment and/or prevention). The animal is, for example, mouse, rat, hamster, rabbit, cat, dog, bovine, sheep, monkey, human, preferably human.

The cell preparation of the present invention can be used in any of the hyperacute phase, acute phase, sub-acute phase, and chronic phase. It can be preferably used for ischemic diseases that are developed acutely, such as acute myocardial infarction and cerebral infarction, especially in the acute and sub-acute phases. The hyperacute phase is a period within 8 hours after onset, and the period is highly likely that tissue cell death can be prevented by stenting, thrombolytic therapy, thrombus removal, and the like. The acute phase is a period of within 8 hours to 2 days after the onset, and the sub-acute phase is a period of within 2 days to 2 weeks after the onset. The chronic phase is a period of not less than 2 weeks after the onset. On the other hand, the cell preparation can be preferably used regardless of time for chronic ischemic diseases that progresses chronically, such as limb ischemia due to chronic circulatory. The administration route is preferably intravenous administration, intraarterial administration, intraportal administration, or topical tissue administration.

The number of cells as a desired component to be administered is not particularly limited. In the intravenous administration, it is, for example, 1×10⁵ cells/kg - 1×10⁹ cells/kg, preferably 1×10⁶ cells/kg - 5×10⁸ cells/kg, particularly preferably 2×10⁸ cells/kg.

In the present invention, a revascularization promoting factor can also be contained. The revascularization promoting factor is not particularly limited and, for example, VEGF, angiopoietin, PDGF, TGF-β, FGF, PlGF, matrix metalloprotease, plasminogen activator and the like can be used, and angiopoietin is preferred. Angiopoietin is a growth factor of glycoprotein that promotes vasculogenesis or angiogenesis. Angiopoietin includes not only 4 kinds of angiopoietin 1 (Ang1), angiopoietin 2 (Ang2), angiopoietin 3 (Ang3), and angiopoietin 4 (Ang4), but also 6 kinds of angiopoietin-related proteins (ANGPTL or angiopoietin-like protein) as similar proteins.

When the cell preparation of the present invention is produced as a preparation for injection, additives generally used in the art can be appropriately used. Examples of the additive include isotonicity agent, stabilizer, buffering agent, preservative, chelating agent, antioxidant and the like. Examples of the isotonicity agent include saccharides such as glucose, sorbitol, mannitol and the like, sodium chloride, glycerol, propylene glycol, polyethylene glycol and the like. Examples of the stabilizer include sodium sulfite and the like. Examples of the buffering agent include borate buffer, phosphate buffer, citrate buffer, tartrate buffer, acetate buffer and the like. Examples of the preservative include para-hydroxybenzoic acid ester, benzyl alcohol, chlorocresol, phenethyl alcohol, benzethonium chloride and the like. Examples of the chelating agent include edetate sodium, sodium citrate and the like. Examples of the antioxidant include sodium sulfite, sodium hydrogen sulfite, sodium ascorbate, sodium thiosulfate and the like.

The production method of the cell preparation of the present invention is not particularly limited as long as it renders the undesired component count/leukocyte count not more than a predetermined value. For example, a mechanical method using an instrument can be used. Specifically, a separation apparatus having a centrifugation means for centrifuging a container with a centrifugation medium and a blood sample injected thereinto, a detection means for detecting undesired components present in the mononuclear cell layer (buffy coat layer which is a layer in which mononuclear cells are present) after centrifugation, a removing means for removing the detected undesired component, and a means for counting the proportion of undesired component count/leukocyte count can be used.

The detecting means is provided with an upper imaging means that images the mononuclear cell layer in the container from the vertically upper direction, a side imaging means that images the mononuclear cell layer in the container from the horizontal direction, and a location information detecting means that detects location information of the undesired component present at the mononuclear cell layer based on the shape information obtained from the images taken by the upper imaging means and the images taken by the side imaging means. As described in he below-mentioned Examples, undesired cell, platelet and aggregate can be distinguished from leukocytes in a normal form based on shape information. The proportion of undesired component count/leukocyte count can be confirmed to be not more than a predetermined value by counting undesired component count/leukocyte count within a certain range.

The screening method of the cell preparation of the present invention has a step of screening for a preparation in which the proportion of leukocytes in a normal form and undesired components (undesired component count/leukocyte count) is not more than a predetermined value. The screening method of the present invention makes it possible to evaluate, before administration of the cell preparation to actual patients with ischemic disease, whether a sufficient therapeutic effect is achieved, and thus, a large effect in the treatment of ischemic disease can be exhibited.

### [Example]

### (Case 1) Disappearance of therapeutic effect of transplantation treatment of myeloid stem cell obtained by specific gravity centrifugation method, due to contamination with many clots, undesired cells, platelets

Bone marrow cells (25 ml) were collected from patients with severe cerebral infarction on day 8 after the onset, a leukocyte cell population containing hematopoietic stem cells derived from bone marrow was separated by the specific gravity centrifugation method using Ficoll-Paque Premium, and a leukocyte suspension containing the isolated hematopoietic stem cells was transvenously administered. The National Institutes of Health Stroke Scale (NIHSS: higher number means higher severity) showing severity of cerebral infarction was 13 points immediately before administration of the cells.

A phase contrast micrograph of the administered cells is shown in Fig. 1. A phase contrast microscope is an optical microscope that can convert the phase difference of light rays into a contrast to allow for observation. It allows noninvasive observation of specimens, and is thus often used particularly when biological cells are observed in detail. For cell observation, a disposable hemocytometer (C-Chip; Neubauer Improved) manufactured by Digital Bio was used, and the cells were observed immediately after injecting the cell suspension into the hemocytometer. As shown in Fig. 1, contamination with components other than leukocytes free of apparent degeneration and expected to provide a therapeutic effect, such as an aggregate (Fig. 1B), undesired cells and platelets (Fig. 1C), was observed. Leukocytes that are expected to provide a therapeutic effect are spheres with a diameter of about 7-25 µm. It is not difficult to distinguish the differences between leukocytes and undesired components by observation using a phase contrast microscope, since the entire image of the cell can be observed by moving the focus of the microscope up and down.

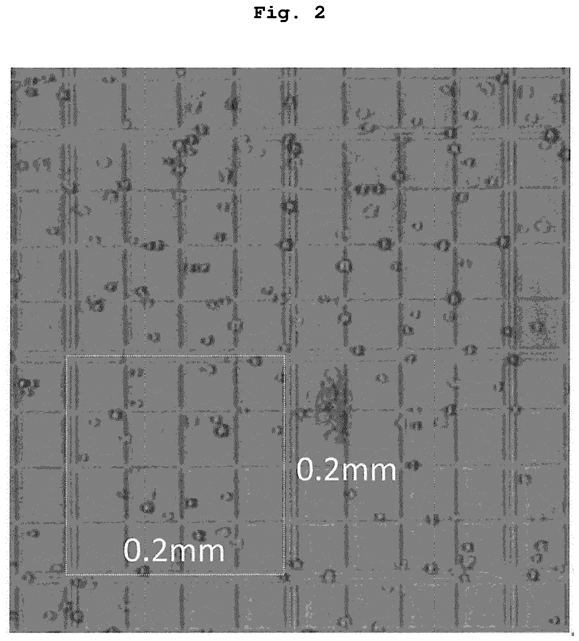

Then, the cell number ratio between leukocytes and undesired cells, and the cell number ratio between leukocytes and platelets were examined (the number of aggregates and the number of platelets were combined and calculated as the number of platelets). As shown in Fig. 2, the method therefor included counting the number of each of leukocytes, undesired cells, and platelets present in square with a side of 0.2 mm on a disposable hemocytometer in 12 fields selected randomly. As a result, the proportion of contamination with undesired cells to the leukocytes, i.e., undesired cell count/leukocyte count was 42.1±6.4% (mean±standard error, hereinafter the same), and similarly, the proportion of contamination with platelets to the leukocytes, i.e., platelet count/leukocyte count was 25.5±5.2%.

In this Case, the number of the administered leukocytes was 2.7x10⁸. In animal experiments using leukocyte population containing hematopoietic stem cells derived from cord blood, CD34-positive cells, which are hematopoietic stem cells, are considered to provide important therapeutic effects (Taguchi et al. J Clin Invest. 2004; 114(3):330-8). The frequency of CD34-positive cells in the administered cells was 1.19%.

NIHSS at discharge from the hospital (1 month after cell administration) was 13 points. The Japan Stroke Scale (JSS: higher number means higher severity) showing other severity of cerebral infarction was 12.76 points. Furthermore, the Barthel Index (BI: higher number means higher daily living faculty) showing the daily living faculty was 20 points.

NIHSS 3 months after the cell administration was 12 points, the degree of improvement was 1 point compared to that immediately before the cell administration, and the degree of improvement was 1 point compared to that at the time of discharge from the hospital. JSS after 3 months from the cell administration was 14.82 points, which was a deterioration of 2.06 points compared with that at the time of discharge from the hospital. BI after 3 months from the cell administration was 15 points, which was a deterioration of 5 points compared with that at the time of discharge from the hospital.

From the above results, it was found that the administration of myeloid stem cells containing a large amount of components other than leukocytes such as clots, undesired cells, platelets, and the like does not provide a sufficient therapeutic effect.

### (Case 2) Demonstration of therapeutic effect by administration of myeloid stem cells obtained by the specific gravity centrifugation method, due to extremely less contamination with clots, undesired cells, platelets

Bone marrow cells (25 ml) were collected from patients with severe cerebral infarction on day 8 after the onset, a leukocyte population containing hematopoietic stem cells derived from bone marrow was separated by the specific gravity centrifugation method using Ficoll-Paque Premium, and a leukocyte suspension containing the isolated hematopoietic stem cells was transvenously administered. NIHSS showing the severity of cerebral infarction was 15 points immediately before administration of the cells.

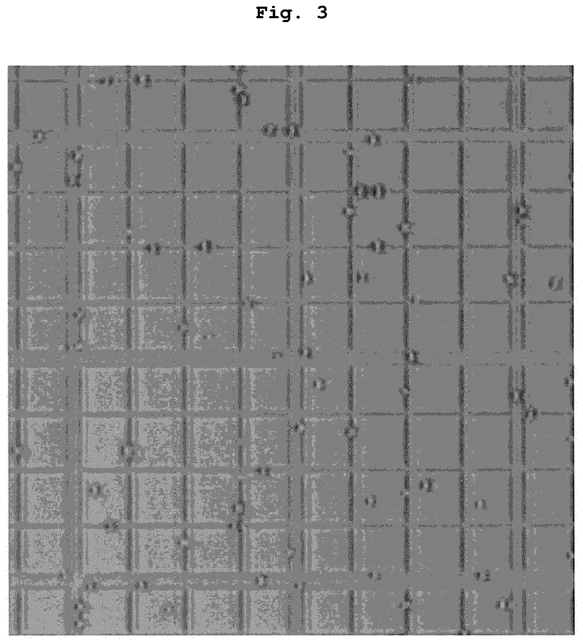

A phase contrast micrograph of the administered cells is shown in Fig. 3. The clot found in Case 1 was not observed, and contamination with undesired cells and platelets was clearly less.

Then, the leukocyte number, undesired cell number, platelet number were counted in 12 fields selected randomly. As a result, the undesired cell count/leukocyte count was 1.8±1.0%, and the platelet count/leukocyte count was 6.5±2.7% (the number of aggregates and the number of platelets were combined and calculated as the number of platelets).

In this Case, the number of the administered leukocytes was 2.4x10⁸. In animal experiments, CD34-positive cells, which are hematopoietic stem cells, are considered to provide important therapeutic effects. However, the frequency of CD34-positive cells in the administered cells was 1.70%.

NIHSS at discharge from the hospital (1 month after cell administration) was 10 points. JSS showing other severity of cerebral infarction was 5.23 points. Furthermore, BI showing the daily living faculty was 30 points.

NIHSS 3 months after the cell administration was 7 points, the degree of improvement was 8 points compared to that immediately before the cell administration, and the degree of improvement was 3 points compared to that at the time of discharge from the hospital. JSS after 3 months from the cell administration was 3.43 points, which was an improvement of 0.80 point compared with that at the time of discharge from the hospital. BI after 3 months from the cell administration was 65 points, which was an improvement of 35 points compared with that at the time of discharge from the hospital.

In this Case, NIHSS immediately before cell treatment was 15 points, and cerebral infarction was severer at the time point before the cell treatment, compared with that in Case 1 (NIHSS immediately before cell treatment was 13 points). Moreover, the number of administered cells was less than that in Case 1.

On the other hand, however, at the time point of 3 months after the cell administration, clearly superior functional prognosis was shown in all evaluations of NIHSS, JSS and BI in this Case than in Cases 1. Furthermore, in Case 1, the effect of neurological functional improvement was not observed in successive observation; however, in this Case, remarkable functional improvement was observed in all evaluations of NIHSS, JSS and BI, immediately before cell administration or after discharge from the hospital to 3 months after the cell administration.

From the above, it was found that the administration of myeloid stem cells containing less amounts of components other than leukocytes such as clots, undesired cells, platelets, and the like provides a sufficient therapeutic effect.

### (Case 3) Demonstration of therapeutic effect by administration of myeloid stem cells obtained by the specific gravity centrifugation method, due to less contamination with clots, undesired cells, platelets

Bone marrow cells (25 ml) were collected from patients with severe cerebral infarction on day 9 after the onset, a leukocyte cell population containing hematopoietic stem cells derived from bone marrow was separated by the specific gravity centrifugation method using Ficoll-Paque Premium, and a leukocyte suspension containing the isolated hematopoietic stem cells was transvenously administered. NIHSS showing severity of cerebral infarction was 17 points immediately before administration of the cells.

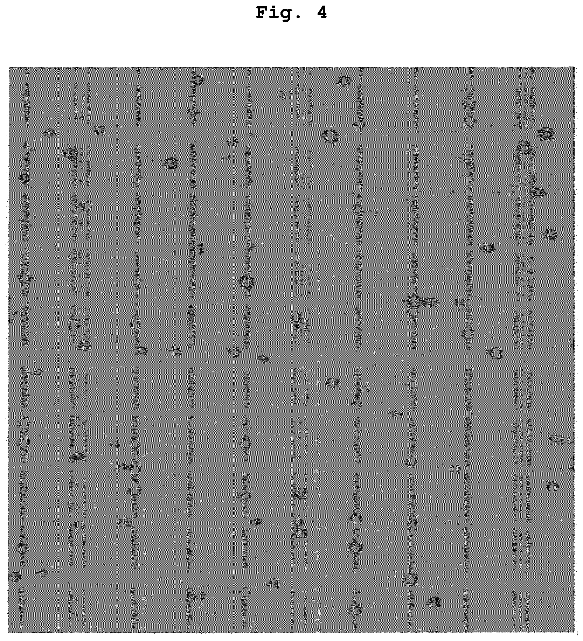

A phase contrast micrograph of the administered cells is shown in Fig. 4. The clot found in Case 1 was not observed, and contamination with undesired cells and platelets was clearly less.

Then, the leukocyte number, undesired cell number, platelet number were counted in 12 fields selected randomly. As a result, the undesired cell count/leukocyte count was 2.8±1.6%, and the platelet count/leukocyte count was 7.8±1.8% (the number of aggregates and the number of platelets were combined and calculated as the number of platelets). From the results of Case 3, it was found that the undesired component count/leukocyte count ≤14.0 (4.4%+9.6%)% is preferable. When error is not considered, the undesired component count/leukocyte count = 10.6(2.8%+7.8%)%, and the undesired component count/leukocyte count ≤10.6% is also preferable.

In this Case, the number of the administered leukocytes was 2.7x10⁸. In animal experiments, CD34-positive cells, which are hematopoietic stem cells, are considered to provide important therapeutic effects. The frequency of CD34-positive cells in the administered cells was 0.66%.

NIHSS at discharge from the hospital (1 month after cell administration) was 12 points. JSS showing other severity of cerebral infarction was 5.11 points. Furthermore, BI showing the daily living faculty was 10 points.

NIHSS 3 months after the cell administration was 9 points, the degree of improvement was 8 points compared to that immediately before the cell administration, and the degree of improvement was 3 points compared to that at the time of discharge from the hospital. JSS after 3 months from the cell administration was 4.08 points, which was an improvement of 1.03 point compared with that at the time of discharge from the hospital. BI after 3 months from the cell administration was 65 points, which was an improvement of 55 points compared with that at the time of discharge from the hospital.

In this Case, NIHSS immediately before cell treatment was 17 points, and cerebral infarction was severer at the time point before the cell treatment, compared with that in Case 1 (NIHSS immediately before cell treatment was 13 points). Moreover, the number of administered CD34 positive cells used as the active ingredient in animal experiments was apparently less than that in Case 1.

On the other hand, however, at the time point of 3 months after the cell administration, clearly superior functional prognosis was shown in all evaluations of NIHSS, JSS and BI in this Case than in Cases 1. Furthermore, in Case 1, the effect of neurological functional improvement was not observed in successive observation; however, in this Case, remarkable functional improvement was observed in all evaluations of NIHSS, JSS and BI, immediately before cell administration or after discharge from the hospital to 3 months after the cell administration.

From the above, it was found that the administration of myeloid stem cells containing less amounts of components other than leukocytes such as clots, undesired cells, platelets, and the like provides a sufficient therapeutic effect.

### (Example 1) Decreased contamination with undesired cells and demonstration of therapeutic effect

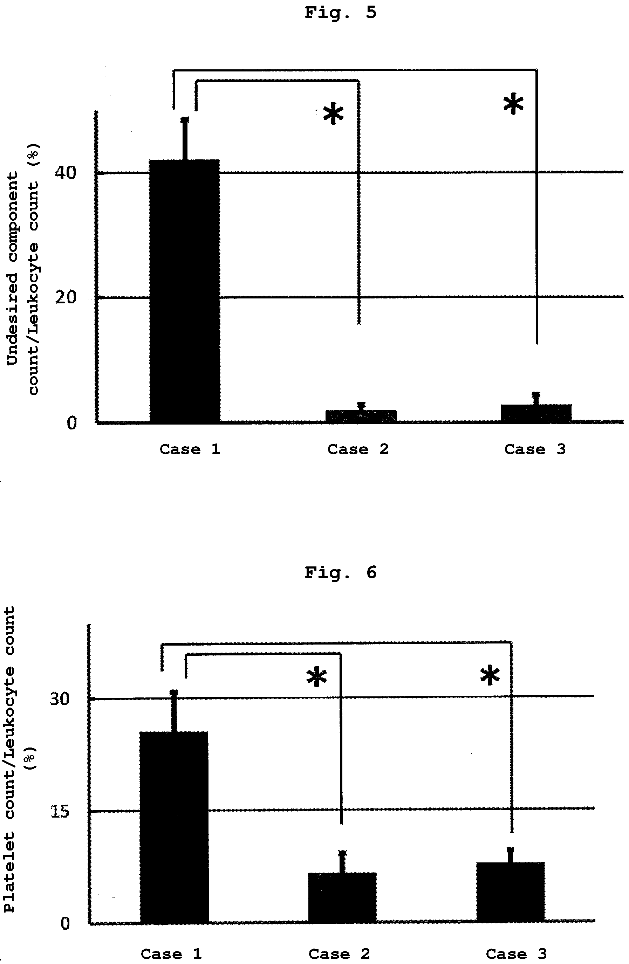

A comparative study was performed regarding the degree of contamination with undesired cells in Cases 1, 2 and 3. The proportions (%) of the undesired cell count/leukocyte count in each Case are shown in Fig. 5. In statistical analysis (variance analysis) using JMP software (manufactured by SAS), it was revealed that the contamination with undesired cells was statistically significantly small in Case 2 and Case 3 in which a therapeutic effect was observed as compared to Case 1 in which no therapeutic effect was observed. From the results of Fig. 5, it is understood that the proportion of leukocytes in a normal form and undesired cells is preferably undesired cell count/leukocyte count ≤20.0%. A comparison between Case 2 and Case 3 reveals that the neurological recovery was better in Case 2 and the contamination proportion with the undesired cells was not statistically significant, but the value was lower in Case 2.

### (Example 2) Decreased contamination with platelets and demonstration of therapeutic effect

A comparative study of the level of contamination with platelets in Cases 1, 2 and 3 was performed. The proportion (%) of platelet count/leukocyte count is shown in Fig. 6 (the number of aggregates and the number of platelets were combined and calculated as the number of platelets). In statistical analysis, it was revealed that the contamination with platelets was statistically significantly small in Case 2 and Case 3 in which a therapeutic effect was observed as compared to Case 1 in which no therapeutic effect was observed. From the results of Fig. 6, it is understood that the proportion of leukocytes in a normal form and platelets is preferably platelet count/leukocyte count ≤15.0%. From the results of Example 1 and Example 2, it was shown that the proportion of leukocytes in a normal form and undesired components in the cell preparation of the present invention is preferably undesired component count/leukocyte count ≤35.0%. A comparison between Case 2 and Case 3 reveals that the neurological recovery was better in Case 2 and the contamination proportion with the platelets was not statistically significant, but the value was lower in Case 2.

### (Example 3) Study of brain regeneration promoting effect of bone marrow-derived leukocyte suspension due to difference in undesired component proportion

An influence of a difference in the undesired component proportion on the brain regeneration promoting effect of bone marrow-derived leukocyte suspension was verified using a cerebral infarction model mouse. The cerebral infarction model mouse was prepared by the following method. 8-Week-old SCID mouse (severe combined immunodeficiency mouse) was fully anesthetized using halothane, and the basicranium was drilled by about 1.5 mm so that the left middle cerebral artery could be directly reached by approaching from the left zygomatic region. The left middle cerebral artery immediately after passing through the olfactory tract (distal side of the olfactory crossing section) was coagulated using a bipolar cautery knife and cut after coagulation, thus permanently occluding the left middle cerebral artery. This cerebral infarction model mouse is superior in the reproducibility of the ischemic site and ischemic intensity localized to the cortex of the left middle cerebral artery region. Human bone marrow fluid was purchased from Lonza, and leukocytes were separated by a specific gravity centrifugation method using Ficoll-Paque PREMIUM. The leukocyte suspension with suppressed contamination with undesired components was prepared only from the buffy coat layer obtained after specific gravity centrifugation. The leukocyte suspension allowing contamination with undesired components concurrently contained leukocytes in the buffy coat layer and cells and the like present in the upper and lower layers of buffy coat layer (upper layer of buffy coat layer being plasma fraction layer [rich in platelets] and lower layer being Ficoll-Paque PREMIUM layer [erythrocytes partially exist]). Using a phase contrast microscope, the degree of contamination with undesired components was counted. As a result, undesired component count/leukocyte count was 25±12% in the leukocyte suspension with suppressed contamination with undesired components, and 100% or more in the leukocyte suspension that allowed contamination with undesired components. Since erythrocyte count/leukocyte count was 0%±0%, platelet count/leukocyte count was 25±12%. From the results, it was found that the undesired component count/leukocyte count ≤37.0 (25%+12%)% is preferable. In addition, undesired component count/leukocyte count ≤25.0% is also preferable.

After 48 hr from the preparation of the cerebral infarction model mouse, saline, a leukocyte suspension (containing desired cells at 1x10⁵) with suppressed contamination with undesired components, or a leukocyte suspension (containing desired cells at 1x10⁵) allowing contamination with undesired components was intravenously administered (each group n=9). One month after leukocyte suspension administration, the brain was removed from the mouse, an overhead view image of the whole brain was taken, and the proportion (%) of [area of brain on which cerebral infarction was created]/[area of normal side on which cerebral infarction was not created] was examined. The results are shown in Fig. 7. Compared to the saline administration group, the regeneration of the brain tissue was promoted in the group of mice administered with the leukocyte suspension with suppressed contamination with undesired components, whereas no significant regeneration promoting effect was observed in the group of mice administered with the leukocyte suspension allowing contamination with undesired components.

### (Example 4) Study of effect of repair of damage due to ischemia (brain regeneration promoting effect) and influence of contamination with extracellular components on inflammatory response in ischemic peripheral region (outside the cerebral infarct area)

The cause of a decrease on the demonstration of effect of repairing damage caused by ischemia (brain regeneration promoting effect) was investigated. The present inventor found that brain regeneration is inhibited by inducing an inflammatory response that is detrimental to the regeneration process, in the ischemic peripheral region (data not shown). Thus, activation of microglia/macrophage in the ischemic peripheral region (emergence and inflammatory response of CD11b positive microglia/macrophage) was studied. A leukocyte suspension with suppressed contamination with undesired components, and a leukocyte suspension allowing contamination with undesired components, which were prepared in Example 3, were administered after 48 hr from operating cerebral infarction in SCID mouse by a method similar to that in Example 3, and induction of an inflammatory response in the ischemic peripheral region (outside the cerebral infarct area) after 24 hr from the administration of the leukocyte suspension was studied by the following method. After 24 hr from the administration of the leukocyte suspension, perfusion fixation was performed using 2% paraformaldehyde fixative, a 20 µm-thick brain section was prepared using vibratome, an inflammatory response was visualized using an anti-CD11b antibody (manufactured by Serotec), and nuclear staining by hematoxylin staining was also performed to clarify the ischemia region. The results are shown in Fig. 8. Fig. 8A shows the brain of a mouse administered with a leukocyte suspension with suppressed contamination with undesired components and after staining with an anti-CD1b antibody, in which CD11b positive microglia/macrophage was scarcely observed in the ischemic peripheral region. Fig. 8B is a stained image of a mouse administered with a leukocyte suspension allowing contamination with undesired components, in which many activated CD1b positive microglia/macrophages were observed in the ischemic peripheral region.

The results of Example 3 and Example 4 show that a leukocyte suspension with suppressed contamination with undesired components (undesired component count/leukocyte count is 25±12%) has a brain tissue regeneration promoting effect and shows a weak activation (inflammatory response) of CD11b positive microglia/macrophage in the ischemic peripheral region, and it was indicated that activation (inflammatory response) of CD11b positive microglia/macrophage in the ischemic peripheral region 72 hr after operating cerebral infarction (24 hr after administration of leukocyte suspension) can be one of the indices of the brain regeneration promoting effect of a leukocyte suspension.

### (Example 5) Study of inflammation inducing effect of mouse leukocyte suspension with undesired component count/leukocyte count of 35%

A mouse leukocyte suspension with the proportion of leukocytes in a normal form and undesired components, i.e., undesired component count/leukocyte count, of 35% was prepared, and activation (inflammatory response) of CD1b positive microglia/macrophage in the ischemic peripheral region (outside the cerebral infarction region) was studied using cerebral infarction model mouse. A leukocyte suspension with undesired component count/leukocyte count of 35% was prepared by the following method. Bone marrow fluid was collected from mouse femur and tibia, leukocytes in the buffy coat layer separated by the specific gravity centrifugation method using Ficoll-Paque PREMIUM, and the cells/components present in the upper and lower layers (plasma fraction layer [rich in platelets] and Ficoll-Paque PREMIUM layer [erythrocytes partially exist]) were isolated, and a leukocyte suspension with undesired component count/leukocyte count of 35% was prepared. For preparation of the leukocyte suspension with the undesired component count/leukocyte count of 35%, leukocyte suspensions with the undesired component count/leukocyte count of not more than and not less than 35% were prepared, appropriately mixed and subjected to the experiment.

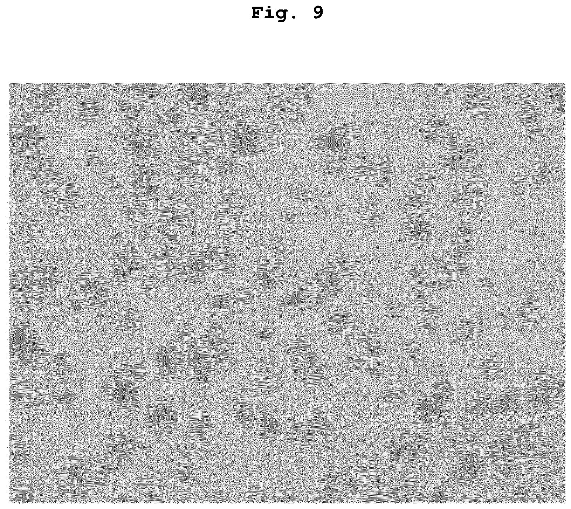

The infarction model mouse used for verification was prepared using 8-week-old SCID mouse and by a method similar to that in Example 3. After 48 hr from operating cerebral infarction, a leukocyte suspension (containing 1x10⁵ desired cells) with undesired component count/leukocyte count of 35% was administered, and the state of activation (inflammatory response) of CD11b positive microglia/macrophage in the ischemic peripheral region (outside the cerebral infarction region) was verified by a method similar to that in Example 4. The results are shown in Fig. 9. As is clear from Fig. 9, it was found that administration of a leukocyte suspension with undesired component count/leukocyte count of 35% did not induce activation (inflammatory response) of CD11b positive microglia/macrophage in the ischemic peripheral region which is considered to inhibit the therapeutic effect on ischemic diseases using leukocytes.

From the above results, it was shown that, when the proportion of leukocytes in a normal form and undesired components (undesired component count/leukocyte count) is ≤35%, activation (inflammatory response) of CD11b positive microglia/macrophage which is considered to inhibit the therapeutic effect on ischemic diseases using leukocytes is not induced, and a therapeutic effect is expected.

### [Industrial Applicability]

The cell preparation of the present invention can be utilized for the treatment and/or prevention of ischemic diseases.

This application is based on a patent application No. 2018-041841 filed in Japan (filing date: March 8, 2018), the contents of which are incorporated in full herein by reference.

## Claims

1. A cell preparation wherein, in a proportion of leukocytes in a normal form and undesired components containing at least one selected from the group consisting of erythrocyte, deformed cell, platelet and aggregate, undesired component count/leukocyte count is not more than a predetermined value.

2. The cell preparation according to claim 1, wherein the undesired component count/leukocyte count is less than or equal to 37.0%.

3. The cell preparation according to claim 1, wherein the undesired component count/leukocyte count is less than or equal to 35.0%.

4. The cell preparation according to claim 1, wherein the undesired component count/leukocyte count is less than or equal to 14.0%.

5. The cell preparation according to any one of claims 1 to 4, wherein the undesired component does not comprise an aggregate.

6. The cell preparation according to any one of claims 1 to 5, wherein the cell preparation is used for the treatment and/or prevention of an ischemic disease.

7. The cell preparation according to claim 6, wherein the ischemic disease is cerebral infarction, myocardial infarction, limb ischemia, renal infarction, pulmonary infarction, splenic infarction, the intestinal infarction, Buerger disease, cerebrovascular dementia, diabetic nephropathy microangiopathy, or diabetic cardiac failure.

8. A method for screening for a cell preparation, comprising a step of screening for a cell preparation in which, in a proportion of leukocytes in a normal form and undesired components containing at least one selected from the group consisting of erythrocyte, deformed cell, platelet and aggregate, undesired component count/leukocyte count is not more than a predetermined value.

9. The screening method according to claim 8, wherein a cell preparation showing undesired component count/leukocyte count of ≤37.0% is screened for in the screening step.

10. The screening method according to claim 8, wherein a cell preparation showing undesired component count/leukocyte count of ≤35.0% is screened for in the screening step.

11. The screening method according to claim 8, wherein a cell preparation showing undesired component count/leukocyte count of ≤14.0% is screened for in the screening step.

12. The screening method according to any one of claims 8 to 11, wherein the undesired component does not comprise an aggregate.

13. The screening method according to any one of claims 8 to 12, wherein a cell preparation used for the treatment and/or prevention of an ischemic disease is screened for in the screening step.

14. The screening method according to claim 13, wherein the ischemic disease is cerebral infarction, myocardial infarction, limb ischemia, renal infarction, pulmonary infarction, splenic infarction, the intestinal infarction, Buerger disease, cerebrovascular dementia, diabetic nephropathy microangiopathy, or diabetic cardiac failure.
